# EUROPEAN PATENT APPLICATION

(11) **EP 0 639 379 A1**
(43) Date of publication of application: **22.02.1995**
(21) Application number: 94830364.9
(22) Date of filing: 18.07.1994
(51) Int. Cl.: A61K 35/78

(54) **A procedure for the preparation of a ginger-root infusion to desensitize teeth and gums and infusion prepared according to said procedure**

(30) Priority: 23.07.1993 IT AN930037
(71) Applicant: Scaloni, Mario, I-60100 Ancona (IT)
(72) Inventor: Scaloni, Mario, I-60100 Ancona (IT)
(74) Representative: Baldi, Claudio

(57) **Abstract**

This invention concerns a procedure for the preparation of a ginger-root infusion for desensitizing teeth and gums to temperature changes and the infusion prepared according to said procedure; this preparation may be used undiluted as a mouthwash or as an additive to toothpaste, ointment, wax etc.

## Description

This patent application concerns a procedure for the preparation of ginger-root infusion for desensitizing teeth and gums to temperature changes, and the infusion obtained according to said procedure.

The infusion in question is developed and produced as a natural medication to eliminate or at least substantially reduce the pain experienced by many people to teeth and gums when drinking very hot or cold drinks or when they eat food that sensitizes teeth or tooth necks.

These painful areas moreover can not be brushed with a normal toothbrush.

To date, the only solution to this problem was to resort to the care of a dental surgeon, often without success (no truly efficient remedy is available) or to use strong chemically based analgesics with harmful side effects.

The infusion according to the invention - which is produced from ginger-root - may be considered as an alternative remedy to the analgesics used to treat the above problems; in view of the fact that it is a natural product, there are no side effects and it may therefore be used without limitation or restrictions.

The action of this preparation causes a total narrowing of the dental tubes, through which teeth and gums are desensitized; the preparation also acts as an antiseptic and astringent for gums.

In particular attention is drawn to the fact that by using this preparation, it will be possible to brush teeth and gums with a normal toothbrush.

In order to develop a ginger-root infusion with the above properties, a procedure consisting of an established sequence of operating phases was perfected after extensive research and experiments.

This procedure involves cutting fine slices of ginger-root, which should preferably be fresh, and then boiling them until half the water placed initially in the container has evaporated; the water in which said slices of ginger-root are boiled is then filtered in order to separate the solid and exploited slices of ginger-root from the infusion.

For major clarity a practical example is given of the preparation according to the invention using 20 gr. of ginger-root.

After cutting the ginger-root in thin slices, this quantity of ginger-root is boiled in half a litre of water for approximately 30/35 minutes: namely the time normally required to boil away 50% of the water used for the infusion; the infusion may be filtered only once half of the water has boiled away.

Using larger quantities of ginger-root will give a more concentrated infusion which is consequently stronger and suitable for more troublesome cases.

The infusion obtained according to then invention may be used undiluted as a mouth wash or as an additive for toothpaste, ointments, wax etc.

Another object of the present invention is a pharmaceutical composition containing a pharmacologically active quantity of the above described ginger infusion in combination with a suitable carrier for the preparation of compositions for topical administration.

For example the active principle can be incorporated in products for the oral hygiene as for example toothpaste, tooth powders, mouthwash, etc.

## Claims

**1)** A procedure for the preparation of a ginger-root infusion to desensitize teeth and gums characterized by the following sequence of preparation phases:
a) thin slicing of ginger-root, which should preferably be fresh;
b) boiling of said thin slices of ginger-root for the time required to boil away approximately half of the water initially placed in the container;
c) filtering of the water in which the ginger-root slices are boiled in order to separate the solid and exploited ginger-root slices from the infusion.

**2)** An infusion of ginger-root obtained according to the procedure described in claim 1).

**3)** Use of a composition according to Claim 2 for the treatment of tooth and gum ache.

**4)** A pharmaceutical composition containing a pharmacologically active quantity of the ginger infusion according to Claim 2 in combination with a suitable carrier for preparing compositions for topical administration.
